# EUROPEAN PATENT SPECIFICATION

(11) **EP 2 073 738 B1**
(45) Date of publication and mention of the grant of the patent: **10.06.2015**
(21) Application number: 07853876.6
(22) Date of filing: 09.10.2007
(51) Int. Cl.: A61B 18/18

(54) **ABLATION CATHETER APPARATUS WITH ONE OR MORE ELECTRODES**
ABLATIONSKATHETERVORRICHTUNG MIT EINER ODER MEHREREN ELEKTRODEN
CATHÉTER D'ABLATION À UNE OU PLUSIEURS ÉLECTRODES

(30) Priority: 19.10.2006 US 551162
(43) Date of publication of application: 01.07.2009
(73) Proprietor: Medwaves, Inc., San Diego, California 92127 (US)
(72) Inventor: ORMSBY, Theodore C., Escondido, California 92029 (US); LEUNG, George L., San Diego, California 92128 (US); SHEN, Gwo Jenn, Carlsbad, California 92009 (US); CHU, Peter, Poway, California 92064 (US)
(74) Representative: Viering, Jentschura & Partner Patent- und Rechtsanwälte
(86) International application number: PCT/US2007/080819
(87) International publication number: WO 2008/051708

(56) References cited:
- US-A- 5 462 545
- US-A- 5 788 692
- US-A- 5 800 494
- US-A- 5 893 885
- US-A1- 2005 059 965
- US-A1- 2006 142 752

## Description

### Background

### 1. Field of the Invention

The present invention generally relates to medical devices used for ablation of biological tissues, and more particularly to an ablation catheter apparatus incorporating one or more electrodes such as electrocardiogram (ECG) electrodes.

### 2. Related Art

Ablation catheters apply energy to a biological tissue site which requires ablation. Such catheters may use various energy modes, such as radiofrequency, ultrasound, laser, cryogenic, and the like. Radio frequency ("RF") ablation catheters generally operate in the microwave frequency range and are used to destroy or ablate biological tissues for therapeutic purposes. In one application, microwave ablation catheters are used to ablate cardiac tissues that cause irregular heartbeats or arrhythmia, avoiding the need for more risky and invasive open heart surgery. In a microwave ablation procedure, the catheter-antenna is passed through the vein for access to the atrium. Within the atrium, the antenna is positioned at the desired location where ablation is required. An intracardiac electrogram is used to identify conductive pathways at the cardiac tissue site that needs to be ablated.

Prior art ablation catheters have been equipped with two or more electrocardiogram ("ECG") electrode rings or buttons made of electrically conductive material to provide the necessary output signal for identification of the desired ablation site. Traditionally, all catheters used for this purpose are installed with metallic electrodes, regardless of energy mode (RF, ultrasound, laser, cryogenic, or the like). Installing metallic electrodes over a microwave antenna has special challenges. Naked metallic electrodes installed wrongly can absorb ablation energy and become hot. Hot electrodes can have adverse effects on the heart or other biological tissues or organs, such as blood clot formation, adherence to tissue, and tissue charring. Naked metallic electrodes can also impede efficient delivery of energy and hinder ablation efficiency. Additionally, metallic electrodes can separate from the catheter when it is bent, resulting in inaccurate or lost signals.

Accordingly, what is needed is an efficient system and method for providing an ECG output signal from an ablation catheter device. US 5,788,692 shows a ablation cather with a plurality of electrode segments. US 2006/ 142752 A1 discloses a further RF ablation catheter apparatus.

### Summary

The present invention is as set out in appended claim 1.

This arrangement avoids the problems of metallic electrodes and also provides electrodes which are of a flexible polymer material which can bend readily with the distal end portion of the catheter as it is shaped or bent to negotiate a path through a body vessel.

Other features and advantages of the present invention will become more readily apparent to those of ordinary skill in the art after reviewing the following detailed description and accompanying drawings.

### Brief Description of the Drawings

The details of the present invention, both as to its structure and operation, may be gleaned in part by study of the accompanying drawings, in which like reference numerals refer to like parts, and in which:
**Figures 1A and 1B** are side elevation views of a shapeable RF ablation catheter according to one embodiment in a straight and bent configuration, respectively;
**Figures 2A and 2B** are side elevation views of a shapeable RF ablation catheter according to another embodiment with a different steering mechanism from Figure 1;
**Figures 3A and 3B** are cross sectional views of the distal end portion of the tip of the catheter of Figure 1 or 2 in a straight configuration and a bent configuration, respectively;
**Figure 4** is a cross-sectional view of the tip or distal end portion of one embodiment of a shapeable or bendable RF ablation catheter incorporating electrodes;
**Figure 5** is a cross-sectional view of the tip or distal end portion of a shapeable or bendable RF ablation catheter having a modified electrode arrangement according to another embodiment;
**Figure 6** is a cross-sectional view of the tip or distal end portion of a shapeable or bendable RF ablation catheter with another electrode arrangement;
**Figure 7** is a cross-sectional view of the tip or distal end portion of a shapeable or bendable RF ablation catheter with a modified electrode arrangement according to another embodiment; and
**Figure 8** is a cross-sectional view of the tip or distal end portion of a shapeable or bendable RF ablation catheter with modified electrodes according to another embodiment.

### Detailed Description

Certain embodiments as disclosed herein provide for systems and methods for ablation of biological tissues in body areas such as the heart, liver, and the like using a bendable radio-frequency (RF) catheter. The catheter is provided with electrodes of a flexible conductive material such as a conductive polymer at its distal end for providing an output signal such as an intracardiac electrocardiogram ("ECG") signal to a control unit to allow physicians to obtain tissue proximity and electrical conductivity information both before and after tissue ablation, as well as to provide other feedback during the ablation procedure.

After reading this description, it will become apparent to one skilled in the art how to implement the invention in various alternative embodiments and alternative applications. However, although various embodiments of the present invention will be described herein, it is understood that these embodiments are presented by way of example only and not limitation. As such, this detailed description of various alternative embodiments should not be construed to limit the scope or breadth of the present invention as set forth in the appended claims.

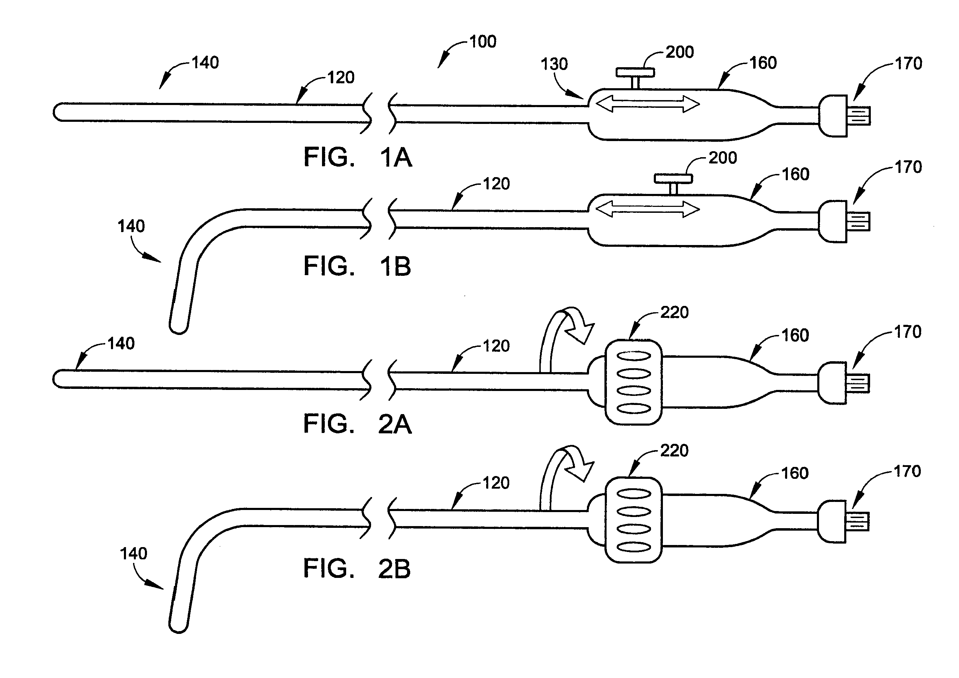

Figures 1A and 1B illustrate a radio-frequency ("RF") ablation catheter system 100 of one embodiment including a shapeable catheter device 100 adapted for insertion into a body vessel of a patient and incorporating an RF antenna for delivering electromagnetic energy to a treatment site, as described in more detail below.

The catheter device 100 has a flexible, elongated tubular body 120 having a proximal portion 130 and a distal or tip portion 140. Located at the proximal portion of the body is a handle chassis 160 containing steering and positioning controls (not illustrated) for the body, activated by actuator 200. In the embodiment of Figures 1A and 1B, the tip portion of the catheter body is activated to bend between the straight configuration of Figure 1A and the bent configuration of Figure 1B by sliding the actuator back and forth in an axial direction. In the modified embodiment of Figures 2A and 2B, the tip portion is bent between the straight and bent configurations by rotating the actuator or collar 220. Suitable mechanisms for controlling bending of the tip portion of catheter body 120 are described in detail in U.S. Patent No. 7,004,938 of Ormsby et al., the contents of which are incorporated herein by reference. However, it will be understood that any suitable mechanism may be incorporated in the catheter device in order to control the bending or steering of the tip portion as it moves through a body vessel, organ, or cavity.

A coupling or electrical connector 170 is provided at the proximal end of the catheter device for connecting the catheter to a control unit or the like containing one or more electronic devices such as an RF generator and controller (not shown) for providing power to the antenna during an ablation procedure. Suitable signal control units are known in the ablation catheter field and are therefore not described in detail here.

The dimensions of the catheter body are adapted as required to suit the particular medical procedure, as is well known in the medical art. In one embodiment, the catheter is used to ablate cardiac tissue. However, the catheter may be used to ablate other types of body tissue in different organs, both internal and external to the body. The tubular body 120 of the catheter device may be generally constructed of a polymer material which is bio-compatible with the body vessel environment. Examples of such materials include thermoplastic elastomer material such as Pebax® available from Autochem Germany, polyethylene, polyurethane, polyester, polyimide, polyamide, and the like, with varying degrees of radiopacity, hardness, and elasticity.

The tubular body of the catheter may be formed with a plurality of segments using one or more of the aforementioned materials or equivalents, such that the catheter body 120 is progressively more flexible towards its distal end. The segments may be joined together by thermal bonding, butt joints, or adhesive bonding. Braiding reinforcement may be provided to the surface of the tubular body to attain a desirable level of stiffness and torsional strength for the catheter to advance and negotiate through the body vessel of the patient, while still allowing the distal end portion to be bent when needed. The distal end portion 140 may be of a softer polymer compound than the remainder of the body, with little or no braiding or reinforcement, to provide the desired flexibility for distal deflection and shaping of the apparatus.

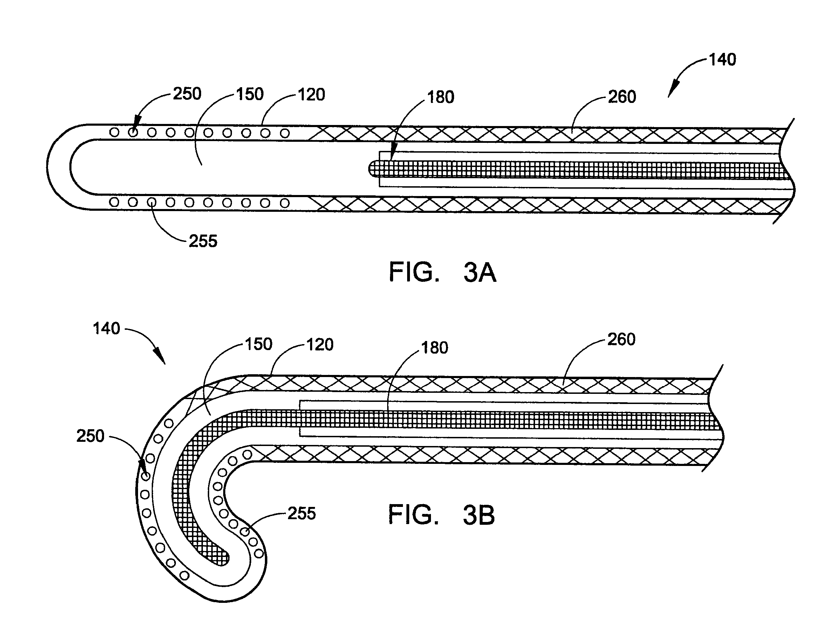

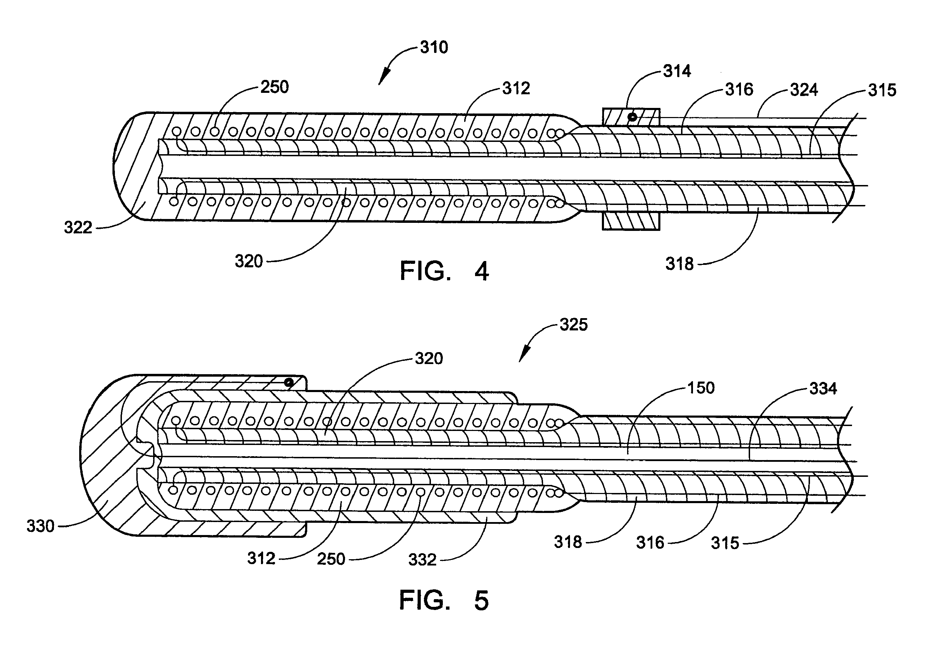

The structure of the catheter in one embodiment will now be described in more detail with reference to Figures 3A and 3B. As noted above, the catheter has a tubular body with a central bore 150 and a closed distal end or tip. The tip may be open in alternative embodiments. In the illustrated embodiment, deflection of the distal end portion of the catheter is accomplished by use of a pre-shaped deflection member 180 which is constrained in a straight orientation in the configuration of Figure 3A and which adopts a bent shape when extended into the bent configuration of Figure 3B. However, it will be understood that other bending or shaping mechanisms may be used in alternative embodiments, as described, for example, in U.S. Patent No. 7,004,938 referenced above. The distal end portion 140 of the tubular body includes an RF antenna 250 comprising a flexible, helically coiled radiating antenna device 255 embedded in the flexible wall of the tubular body, as best illustrated in Figures 3A and 3B. The antenna device can therefore bend as the distal end portion is shaped to conform to a body vessel or the like, as illustrated in Figure 3B. Opposite ends of the antenna device are connected to electrical conductors or leads for connection to the proximal end connector 170 and thereby to a source of RF energy in the catheter control unit (not illustrated), as will be described in more detail below in connection with Figure 4. Other antenna devices may be provided in alternative embodiments, and the diameter, pitch and length of the coiled device 255, and the conductive material used for the device 255, may vary according to the particular procedure and flexibility requirements.

The electrical conductors which connect the RF antenna to the connector 170 may be of a flexible mesh or braided wire construction 260 or of a thin-film electrically conductive material. In the embodiment illustrated in Figures 3A and 3B, the conductors are shown schematically as a mesh construction embedded in the walls of the tubular body 120 of the catheter. In alternative arrangements, separate conductors may be used to provide power to the antenna 250. Figure 4 illustrates the distal end portion 310 of a first embodiment of a modified catheter having integrated electrodes 312, 314. In one embodiment, the electrodes are ECG electrodes, although they may be other types of electrodes in other embodiments. Although two electrodes are illustrated in Figure 4, in other embodiments one electrode or more than two such electrodes may be provided. Some parts of the catheter of Figure 4 are identical to those in Figures 1 to 3 and like reference numerals have been used for like parts, as appropriate. In the embodiment of Figure 4, a pair of coaxial inner and outer tubular conductors 315, 316 extend along the length of the tubular body 318, with the outer conductor 316 connected to the proximal end of RF antenna 250 and the inner conductor 315 connected to the distal end of the RF antenna adjacent the tip of the catheter. The structure of the remainder of the tubular body 318 which is not shown in Figure 4 may be identical to that of tubular body 120 described above, and a similar connector 170 (not illustrated) may be provided at the proximal end of the catheter for connecting the conductors to a suitable RF source. The distal end portion illustrated in Figure 4 will be shapeable or bendable in a similar manner and using the same or similar control devices as were described above in connection with Figures 1 to 3.

In the embodiment of Figure 4, the tubular body 318 is of dielectric material such as a non-conductive polymer and has a portion 320 of reduced outer diameter at its forward end. The first electrode 312 comprises a sleeve of flexible conductive material mounted over the reduced diameter end portion 320 of the tubular body and having an end portion or tip 322 extending over the open end of portion 320. The RF or microwave antenna 250 is embedded in the sleeve or electrode 312. The inner and outer conductors 315, 316 extend through the tubular body 318 as illustrated for connection to the opposite ends of the antenna coil 250. The second electrode 314 comprises a ring of flexible conductive material mounted over the tubular body 318 at a location spaced rearwardly from the rear end of conductive sleeve or electrode 312. The two electrodes may be secured over the inner tubular body 318 by adhesive, bonding, mechanical force, heat sealing or the like. The flexible conductive material forming the electrodes is at least substantially non-metallic material and may be a conductive polymer material which is sufficiently bendable to allow bending of the distal end portion 310 between the positions illustrated in Figures 1A and 1B.

In an alternative embodiment, the electrode ring 314 may be mounted flush in an annular recess or gap in the outer surface of the tubular body, or may be molded integrally with the tubular body, so that it does not project outwardly from the outer surface of the body 318. A conductor or connector 324 extends from electrode ring 314 to the connector 170 at the proximal end of the catheter, for suitable connection to an ECG monitor or the like in a control unit (not illustrated) for the catheter. Conductor 324 is shown spaced from the outer surface of body 318 in Figure 4 for clarity, but may be a line of conductive ink or adhesive over the outer surface of the tubular body, or may alternatively be embedded in the body 318 outside conductor 316, One of the conductors 315 or 316 will also be connected to the ECG or other monitor for suitable monitoring of the signal detected between the two electrodes 312, 314. In one embodiment, both electrodes are of a flexible, conductive polymer material, i.e. a polymer material loaded with conductive materials.

Figure 5 illustrates the distal end portion 325 of a catheter with a modified electrode arrangement in which the electrode ring 314 of Figure 4 is replaced by an electrode end cap 330. Electrodes 312, 330 are of flexible conductive material such as a conductive polymer material as in Figure 4. In this embodiment, the conductive sleeve 312 in which the antenna is mounted has an outer cover layer 332 of non-conductive polymer material extending along at least part of its length and over its distal end, providing a non-conductive shield layer between the first and second electrodes 312, 330. A conductor or connector wire 334 extends from the connector at the proximal end of the catheter through the central lumen 150 of the tubular body 318 and into the electrode end cap 330 to provide a signal path between the electrode and the ECG monitor. The catheter of Figure 5 is otherwise identical to that of the previous embodiment and like reference numerals have been used as appropriate. Conductive sleeve 312, non-conductive layer 332, and end cap 330 may be laminated together over the tubular body 318 by any suitable means such as bonding, heat sealing, adhesive, or the like.

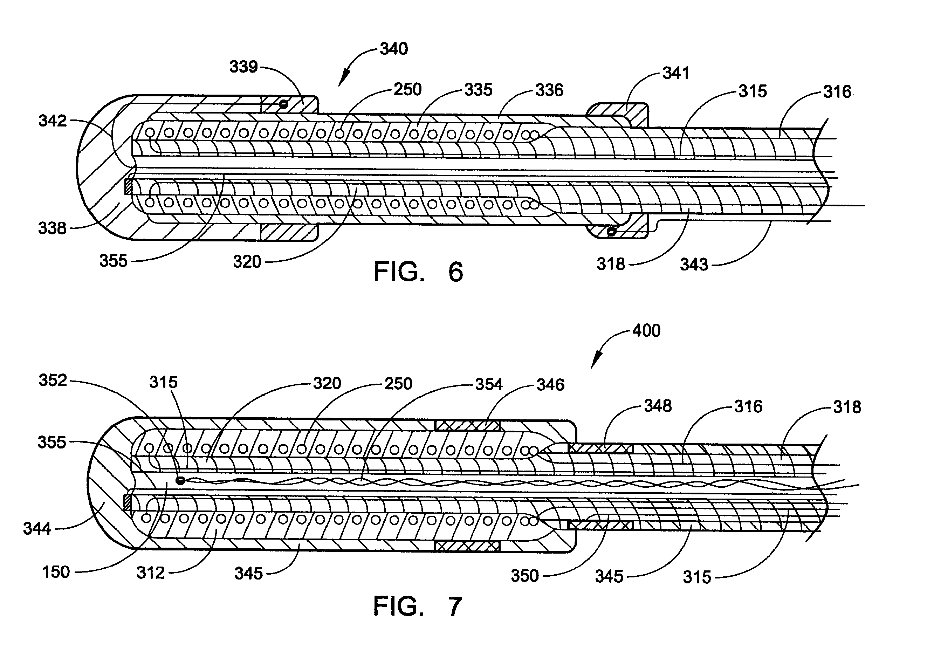

Figure 6 illustrates the distal end portion 340 of a catheter having another modified electrode arrangement. Parts of the cathode of this embodiment are identical to those of Figures 4 and 5 and like reference numerals have been used for like parts as appropriate. Unlike the previous embodiments, the sleeve 335 in which the antenna coil 250 is embedded does not comprise one of the two electrodes. As in the previous embodiment, sleeve 335 is mounted over the reduced diameter end portion 320 of the tubular body 318, which is of dielectric or non-conductive material, and the antenna coil 250 is connected at its opposite ends to the distal ends of the inner and outer conductors 315, 316.

In the embodiment of Figure 6, an outer layer 336 of non-conductive material, such as a non-conductive polymer material, extends over the conductive sleeve 335 and has an end cap portion 338 extending over the tip of the tubular body 318. The electrodes in this embodiment comprise a pair of conductive rings 339, 341 mounted at spaced intervals on the outer, non-conductive layer 336. The ring electrodes may be of conductive polymer material. The first ring 339 is positioned adjacent the non-conductive end cap portion 338 and the second ring 341 is positioned adjacent the rear end of the conductive layer 336. A central conductor or connector wire 342 extends through the hollow central bore or lumen of the tubular body 318, through the non-conductive end cap portion 338, and bends back to terminate in the first conductive ring electrode 339. In one embodiment, the part of connector wire 342 shown extending through lumen 150 may be a line of conductive ink or adhesive on the inner surface of tubular body 318. A second conductor or connector wire 343 extends along the outside of the tubular body 318 and is connected to the second conductive ring electrode 341. It will be understood that the connector wire 343 may comprise a line of conductive ink or adhesive on tubular body 318, or may alternatively be embedded in the tubular body 318 at location spaced outside the outer tubular conductor 316. The various conductive and non-conductive polymer layers of the distal end portion 340, including the electrode rings, are suitably laminated together by heat sealing, adhesive bonding, or the like.

Also shown in Figure 6 is a pull wire 355 which extends through the lumen 150 to the tip 338 and is attached to suitable steering and positioning controls (not illustrated) at the proximal end of the catheter, for controlling bending of the distal end portion. Such a pull wire mechanism is described in U.S. Patent No. 7,004,938 referenced above, the contents of which are incorporated herein by reference. It may be understood that a similar position control mechanism will be provided in the embodiments of Figures 4 to 6, or the mechanism 180 of Figure 3 may be provided in any of these embodiments.

Figure 7 illustrates the distal end portion 400 of a catheter according to another embodiment. Again, some parts of the catheter illustrated in Figure 7 are identical to those of Figures 4 to 6 and like reference numerals have been used as appropriate. As in the previous embodiment, a tubular body 318 of flexible dielectric material extends the length of the catheter and has a central through bore or lumen 150 and an end portion 320 of reduced outer diameter over which the sleeve 312 containing embedded RF antenna 250 is mounted. As in the previous embodiments, sleeve 312 is of conductive polymer material and the ends of the antenna are connected to the distal end connector 170 (Figure 1) of the catheter by means of inner and outer cylindrical conductors 315, 316 extending through the tubular body 318, in the manner described above in connection with Figure 1. Unlike the previous embodiments, an outer cover layer 345 of non-conductive polymer material extends along the entire length of the catheter, over the tubular body 318 and sleeve 312, and has a forward end or tip 344 covering the forward end of the sleeve and tubular body. A pair of contact rings 346,348 are mounted in the outer cover layer 345 in the distal end portion of the catheter, with the forward contact ring 346 located over the sleeve 312 and in electrical contact with the sleeve, and the rear contact ring 348 located slightly rearwardly from sleeve 312. Each ring is of a flexible conductive material such as conductive polymer material. Rings 346,348 and outer cover layer 345 are suitably bonded together and laminated over the tubular body 318 and conductive polymer sleeve 312.

The forward contact ring 346 is connected to the proximal end connector 170 via the conductive sleeve 312 and the outer conductor 316 which also provides power to the antenna 250. The rear contact ring 348 is connected to a conductive wire 350 which extends through the tubular body 318 to the proximal end connector 170 of the catheter. The conductors 316, 350 therefore provide the output for the ECG monitor in the control unit in this embodiment.

The embodiment of Figure 7 also includes a temperature sensor 352 in the lumen 150 adjacent the tip of the catheter. In the illustrated embodiment, the temperature sensor 352 may be a thermistor, thermocouple, or the like and has a thermocouple junction or sensor end 352 and a pair of braided wires or conductors 354 extending from the sensor 352 through the tubular body to the connector 170 at the proximal end of the catheter, where they are connected to control circuitry for monitoring the temperature at the distal end of the catheter and controlling the antenna operation. A pull wire 355 is attached to the tip 344 of the catheter and extends through the central lumen 150 through the length of the catheter for attachment to a suitable steering and control mechanism (not illustrated), as in the previous embodiment.

A system for monitoring and controlling operation of an RF ablation catheter incorporating a temperature sensor is described in co-pending application Serial No. 11/479,259 filed on June 30, 2006, the contents of which are incorporated herein by reference. It will be understood that a similar control system may be provided for controlling operation of the microwave antenna in this embodiment or other embodiments described above, with suitable inclusion of a temperature sensor.

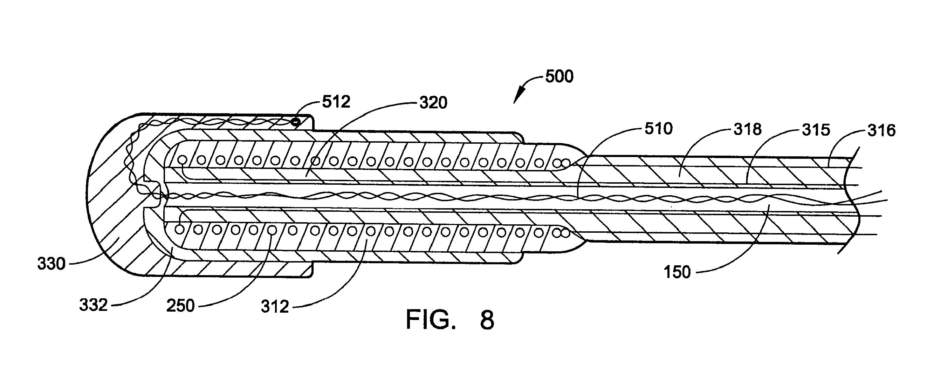

Figure 8 illustrates a modification of the embodiment of Figure 5, and like reference numerals are used for like parts as appropriate. In this embodiment, as in the previous embodiments, a tubular body 318 of dielectric material having a central lumen 150 extends the entire length of the catheter, and has a reduced outer diameter portion 320 at the distal end portion 500 of the catheter. Conductive sleeve 312 is mounted over the portion 320 and the RF antenna 250 is embedded in sleeve 312. As in the embodiment of Figure 5, the electrodes comprise the conductive sleeve 312 and a conductive tip 330 mounted over the end of the catheter, with a layer 332 of non-conductive material such as non-conductive polymer between the electrodes 312 and 330. The various layers of conductive and non-conductive materials in the embodiment of Figure 8 will also be laminated together by any suitable means such as heat, adhesives and mechanical force.

In Figure 8, the conductive wire 334 which is connected to the conductive tip electrode 330 of Figure 5 is eliminated, and is replaced with double thermocouple wires 510 which extend through lumen 150 from the proximal end connector 170 of the catheter and into the conductive tip electrode 330, with a thermocouple junction 512 at the end of the double wires providing a temperature sensor. The thermocouple wires therefore have the dual function of providing a temperature sensor output as well as providing an ECG monitor output in combination with outer antenna conductor 316. The ECG output may be measured between conductor 316 and either one of the thermocouple wires 510. The temperature output may be used in monitoring and controlling operation of the RF antenna, as described above in connection with Figure 7.

In each of the embodiments of Figures 4 to 8, electrodes are mounted at the distal end portion of a shapeable or bendable catheter to allow physicians to locate a tissue region causing problems and to obtain both optimum tissue proximity and electrical conductive activities before and after ablation, as well as to obtain feedback of their actions. Although two electrodes are provided in these embodiments, only one electrode or more than two electrodes may be provided in other embodiments. The electrode or electrodes in these embodiments may be ECG or other types of electrodes. Radio-opaque markers (not illustrated) at the distal end portion of the catheter may also be used to aid in positioning the tip of the catheter, as is known in the field. Where the electrodes are ECG electrodes, it will be understood that the conductor wires connected to the electrodes and to the proximal end connector 170 of the catheter will communicate with an external ECG system and monitor (not illustrated) via a suitable connection cable which will transmit ECG signals between the electrodes and ECG system. The antenna conductors and thermocouple wires (if a temperature sensor is present) will be similarly connected to an appropriate antenna output control system.

In each of the above embodiments, the RF antenna 250 is adapted to receive and radiate electromagnetic energy in order to treat a selected biological tissue site. An example of a suitable spectrum of radio frequency energy for use in the ablation catheter is that of the microwave frequency range above 300 MHz. The RF antenna is capable of applying substantially uniformly distributed electromagnetic field energy along the RF antenna in a direction substantially normal to the longitudinal axis of antenna 250.

The electrodes in the embodiments of Figures 4 to 8 are made of a suitable flexible conductive material, so that they can bend with the remainder of the distal end portion during steering. Such electrodes avoid or reduce the problems encountered with metallic electrodes, since they do not absorb microwave energy to any great extent and do not become excessively hot. The electrodes may be of an at least substantially non-metallic material, and in one embodiment they are made from a conductive polymer material such as nylon, polyethylene, polyolefin, polypropylene, polycarbonate, Pebax ®, TPE (thermoplastic elastomers) and blends, loaded with a selective conductive material. Other non-conductive parts of the catheter may be of the same polymer material or different polymer materials. The conductive material may be micro-carbon spheres, carbon particles, carbon nanotubes, nickel dust, or the like. The electrodes may be made entirely of conductive polymer material or may be a mixture of conductive and non-conductive polymer material, or a mixture of conductive and non-conductive materials with metal substrates. The composite polymer material is selected to have a relatively low resistance for reduced interference with the microwave radiation pattern, and to be hydrophilic for improved wetability on the outer surface of the catheter.

Communication between the electrodes and the connector 170 at the proximal end of the catheter may be provided in some embodiments by means of conductive ink or adhesive applied over the polymer surface. For example, conductor 324 of Figure 4 or conductor 342 of Figure 6 may be a line of conductive ink or adhesive over the outer surface of the tubular body 318 extending from electrode ring 314 to the proximal end of the catheter. Conductor 350 of Figure 7 may be a line of conductive ink or adhesive over the outer surface of non-conductive tubular body 318, with the outer layer 345 of non-conductive polymer laminated over the tubular body and conductor line 350.

Heat energy, adhesives, and/or mechanical force may be used to laminate the conductive and non-conductive polymer layers in the embodiments of Figures 4 to 8. Metallic substrates may also be laminated between the polymer layers, such as the inner and outer tubular conductors which provide power for operating RF antenna 250.

The above description of the disclosed embodiments is provided to enable any person skilled in the art to make or use the invention. Various modifications to these embodiments will be readily apparent to those skilled in the art, and the generic principles described herein can be applied to other embodiments without departing from the scope of the invention. Thus, it is to be understood that the description and drawings presented herein represent a presently preferred embodiment of the invention and are, therefore, representative of the subject matter which is broadly contemplated by the present invention. It is further understood that the scope of the present invention fully encompasses other embodiments and that the scope of the present invention is accordingly limited by nothing other than the appended claims.

## Claims

1. An RF ablation catheter apparatus, comprising:
an elongate catheter (100) adapted for insertion into a body vessel of a patient, the catheter (100) having a proximal end (130) and a distal end (140) portion, at least the distal end (140) portion of the catheter (100) being flexible for allowing the distal end (140) portion of the catheter (100) to be deflected;
a radio-frequency, "RF", antenna (250) disposed at the distal end (140) portion of the catheter (100) and adapted to receive input RF energy for the ablation of biological tissue;
an electrical connector (170) at the proximal end (130) of the catheter (100) for connection to a power supply for the RF antenna (250);
at least one electrode (312, 314) disposed at the distal end (140) portion of the catheter (100) and electrically coupled to the connector (170) at the proximal end (130) of the catheter (100) for connection to a monitor;
the electrode (312) being of a flexible, electrically conductive material;
the conductive material of the electrode (312) is constructed of a flexible polymer material loaded with a conductive material;
the electrode (312) comprising an elongate electrode sleeve at the distal end (140) portion of the catheter (100) ; and
the antenna (250) is embedded in said electrode sleeve.

2. The apparatus of claim 1, wherein said at least one electrode (314) is an electrocardiogram, "ECG", electrode.

3. The apparatus of claim 1 or 2, wherein the polymer is selected from the group consisting of polyethylene, polyolefin, polypropylene, polycarbonate, nylon and thermoplastic elastomer material.

4. The apparatus of claim 1, wherein the conductive material of said electrode (312, 314) is selected from the group consisting of micro-carbon spheres, carbon particles, carbon nanotubes, and nickel dust.

5. The apparatus of claim 1, further comprising at least one electrical conductor (150) extending through the catheter (100) and coupled at a first end to said antenna (250) and to said proximal end connector (170) at a second end.

6. The apparatus of any preceding claim, further comprising a second electrode (314) of flexible, electrically conductive material spaced from said elongate electrode sleeve (312).

7. The apparatus of claim 6, wherein the catheter (100) comprises a tubular body (318) and the second electrode (314) comprises a ring mounted on said tubular body (318).

8. The apparatus of claim 1, wherein said antenna (250) comprises a helical coil embedded in said electrode sleeve and at least one electrical conductor for connecting said helical coil to said RF power source to provide an electrical connection from said electrode sleeve to said proximal end connector (170).

9. The apparatus of claim 1, further comprising a temperature sensor (352, 512) mounted at the distal end (140) portion of the catheter (100) and conductor means extending through the catheter (100) to said proximal end (170) connector and coupled to said temperature sensor (352, 512).

10. The apparatus of claim 9, wherein said temperature sensor (352, 512) is coupled to said second electrode (314) and said conductor means comprises a pair of conductors (339, 341) one of which further comprises the electrical coupling from the second electrode to the proximal end connector (170).

11. The apparatus of claim 10, wherein said second electrode (314) comprises an end cap (330) at the distal end (140) portion of the catheter (100), and said temperature sensor (512) is embedded in said end cap (330).

12. The apparatus of claim 1, further comprising a deflection member (180) adapted to control deflection of said distal end (140) portion of the catheter (100).

13. The apparatus of claim 1, further comprising a pair of inner and outer coaxial electrical conductors (315, 316) electrically coupling said catheter (100) from said RF antenna (250) to said electrical connector (170).

## Patentansprüche

1. Eine RF-Ablationskathetervorrichtung, aufweisend:
einen langgestreckten Katheter (100), welcher zum Einführen in ein Körpergefäß eines Patienten eingerichtet ist, wobei der Katheter (100) ein proximales Ende (130) und einen distalen Endabschnitt (140) aufweist, wobei zumindest der distale Endabschnitt (140) des Katheters (100) flexibel ist, um es dem distalen Endabschnitt (140) des Katheters (100) zu ermöglichen, umgelenkt zu werden,
eine Radiofrequenz -(RF)- Antenne (250), welche an dem distalen Endabschnitt (140) des Katheters (100) angeordnet ist, und eingerichtet ist zum Empfangen von Radiofrequenz-Energie zur Ablation von biologischem Gewebe,
einen elektrischen Verbinder (170) an dem proximalen Ende (130) des Katheters (100) zum Verbinden der RF-Antenne (250) mit einer Energieversorgung,
mindestens eine Elektrode (312, 314), welche an dem distalen Endabschnitt (140) des Katheters (100) angeordnet ist und zum Verbinden mit einem Monitor elektrisch an den Verbinder (170) an dem proximalen Ende (130) des Katheters (100) gekuppelt ist, wobei die Elektrode (312) aus einem flexiblen, elektrisch leitfähigem Material ist,
wobei das leitfähige Material der Elektrode (312) aus einem flexiblen Polymermaterial konstruiert ist, welches mit leitfähigem Material bestückt ist,
wobei die Elektrode (312) an dem distalen Endabschnitt (140) des Katheters (100) eine langgestreckte Elektrodenhülse aufweist, und wobei die Antenne (250) in die Elektrodenhülse eingebettet ist.

2. Vorrichtung gemäß Anspruch 1, wobei die mindestens eine Elektrode (314) eine Elektrokardiogramm -(EKG)-Elektrode ist.

3. Vorrichtung gemäß Anspruch 1 oder 2, wobei das Polymer ausgewählt ist aus der Gruppe bestehend aus Polyethylen, Polyolefin, Polypropylen, Polycarbonat, Nylon und thermoplastischem Elastomermaterial.

4. Vorrichtung gemäß Anspruch 1, wobei das leitfähige Material der Elektrode (312, 314) ausgewählt ist aus der Gruppe bestehend aus Mikro-Kohlenstoffkugeln, Kohlenstoffpartikeln, Kohlenstoff-Nanoröhren und Nickelstaub.

5. Vorrichtung gemäß Anspruch 1, ferner aufweisend mindestens einen elektrischen Leiter (150), welcher sich durch den Katheter (100) erstreckt und an einem ersten Ende an die Antenne (250) und an einem zweiten Ende an den Verbinder an dem proximalen Ende (170) gekuppelt ist.

6. Vorrichtung gemäß irgendeinem der vorangegangenen Ansprüche, ferner aufweisend eine zweite Elektrode (314) aus flexiblem, elektrisch leitfähigem Material, welche in einem Abstand zu der langgestreckten Elektrodenhülse (312) angeordnet ist.

7. Vorrichtung gemäß Anspruch 6, wobei der Katheter (100) einen rohrförmigen Körper (318) aufweist und die zweite Elektrode (314) einen Ring aufweist, welcher an dem rohrförmigen Körper (318) montiert ist.

8. Vorrichtung gemäß Anspruch 1, wobei die Antenne (250) eine wendelförmige Spule aufweist, welche in die Elektrodenhülse eingebettet ist, und mindestens einen elektrischen Leiter zum Verbinden der wendelförmigen Spule mit der RF-Energiequelle, um eine elektrische Verbindung von der Elektrodenhülse zu dem Verbinder an dem proximalen Ende (170) bereitzustellen.

9. Vorrichtung gemäß Anspruch 1, ferner aufweisend einen Temperatursensor (352, 512), welcher an dem distalen Endabschnitt (140) des Katheters (100) montiert ist, und ein Leitermittel, welches sich durch den Katheter (100) zu dem Verbinder an dem proximalen Ende (170) erstreckt und mit dem Temperatursensor (352, 512) verbunden ist.

10. Vorrichtung gemäß Anspruch 9, wobei der Temperatursensor (352, 512) mit der zweiten Elektrode (314) gekuppelt ist, und das Leitermittel ein Paar von Leitern (339, 341) aufweist, von denen einer ferner die elektrische Kupplung von der zweiten Elektrode zu dem Verbinder an dem proximalen Ende (170) aufweist.

11. Vorrichtung gemäß Anspruch 10, wobei die zweite Elektrode (314) an dem distalen Endabschnitt (140) des Katheters (100) eine Endkappe (330) aufweist, und der Temperatursensor (512) in die Endkappe (330) eingebettet ist.

12. Vorrichtung gemäß Anspruch 1, ferner aufweisend ein Umlenkelement (180), welches eingerichtet ist, das Umlenken des distalen Endabschnitts (140) des Katheters (100) zu steuern.

13. Vorrichtung gemäß Anspruch 1, ferner aufweisend ein Paar von einem inneren und einem äußeren koaxialen, elektrischen Leiter (315, 316), welche den Katheter (100) von der RF-Antenne (250) zu dem elektrischen Verbinder (170) elektrisch kuppeln.

## Revendications

1. Appareil à cathéter d'ablation RF, comprenant :
un cathéter (100) allongé adapté pour une insertion dans un vaisseau du corps d'un patient, le cathéter (100) comportant une partie d'extrémité proximale (130) et une partie d'extrémité distale (140), au moins la partie d'extrémité distale (140) du cathéter (100) étant souple pour permettre la déflexion de la partie d'extrémité distale (140) du cathéter (100) ;
une antenne radiofréquence, « RF », (250) disposée au niveau de la partie d'extrémité distale (140) du cathéter (100) et adaptée pour recevoir une énergie RF d'entrée pour l'ablation d'un tissu biologique ;
un connecteur électrique (170) à l'extrémité proximale (130) du cathéter (100) pour une connexion à une alimentation pour l'antenne RF (250) ;
au moins une électrode (312, 314) disposée au niveau de la partie d'extrémité distale (140) du cathéter (100) et couplée électriquement au connecteur (170) à l'extrémité proximale (130) du cathéter (100) pour une connexion à un moniteur ;
l'électrode (312) étant en un matériau électriquement conducteur souple ;
le matériau conducteur de l'électrode (312) étant composé d'un matériau polymérique souple chargé avec un matériau conducteur ;
l'électrode (312) comprenant un manchon d'électrode allongé au niveau de la partie d'extrémité distale (140) du cathéter (100) ; et
l'antenne (250) étant logée dans ledit manchon d'électrode.

2. Appareil selon la revendication 1, dans lequel ladite au moins une électrode (314) est une électrode d'électrocardiogramme, « ECG ».

3. Appareil selon la revendication 1 ou 2, dans lequel le polymère est sélectionné dans le groupe consistant en le polyéthylène, une polyoléfine, le polypropylène, le polycarbonate, le nylon et un matériau élastomère thermoplastique.

4. Appareil selon la revendication 1, dans lequel le matériau conducteur de ladite électrode (312, 314) est sélectionné dans le groupe consistant en des microsphères de carbone, des particules de carbone, des nanotubes de carbone, et de la poussière de nickel.

5. Appareil selon la revendication 1, comprenant en outre au moins un conducteur électrique (150) s'étendant à travers le cathéter (100) et couplé à une première extrémité à ladite antenne (250) et audit connecteur d'extrémité proximale (170) à une deuxième extrémité.

6. Appareil selon l'une quelconque des revendications précédentes, comprenant en outre une deuxième électrode (314) en un matériau électriquement conducteur souple espacée dudit manchon d'électrode (312) allongé.

7. Appareil selon la revendication 6, dans lequel le cathéter (100) comprend un corps tubulaire (318) et la deuxième électrode (314) comprend une bague montée sur ledit corps tubulaire (318).

8. Appareil selon la revendication 1, dans lequel ladite antenne (250) comprend une bobine hélicoïdale logée dans ledit manchon d'électrode et au moins un conducteur électrique pour connecter ladite bobine hélicoïdale à ladite source de puissance RF pour réaliser une connexion électrique dudit manchon d'électrode audit connecteur d'extrémité proximale (170).

9. Appareil selon la revendication 1, comprenant en outre un capteur de température (352, 512) monté au niveau de la partie d'extrémité distale (140) du cathéter (100) et des moyens conducteurs s'étendant à travers le cathéter (100) vers ledit connecteur d'extrémité proximale (170) et couplés audit capteur de température (352, 512).

10. Appareil selon la revendication 9, dans lequel ledit capteur de température (352, 512) est couplé à ladite deuxième électrode (314) et lesdits moyens conducteurs comprennent une paire de conducteurs (339, 341) dont l'un comprend en outre le couplage électrique de la deuxième électrode au connecteur d'extrémité proximale (170).

11. Appareil selon la revendication 10, dans lequel ladite deuxième électrode (314) comprend un capuchon d'extrémité (330) au niveau de la partie d'extrémité distale (140) du cathéter (100), et ledit capteur de température (512) est logé dans ledit capuchon d'extrémité (330).

12. Appareil selon la revendication 1, comprenant en outre un élément de déflexion (180) adapté pour commander la déflexion de ladite partie d'extrémité distale (140) du cathéter (100).

13. Appareil selon la revendication 1, comprenant en outre une paire de conducteurs électriques coaxiaux (315, 316) intérieur et extérieur couplant électriquement ledit cathéter (100) de ladite antenne RF (250) audit connecteur électrique (170).
